Europäisches Patentamt

European Patent Office

Office européen des brevets

(19)

(11) Veröffentlichungsnummer: **0 392 369**

**A1**

(12) **EUROPÄISCHE PATENTANMELDUNG**

(21) Anmeldenummer: **90106568.0**

(22) Anmeldetag: **05.04.90**

(51) Int. Cl.5: **C12P 21/08, G01N 33/573, C07K 7/04**

(30) Priorität: **11.04.89 DE 3911794**

(43) Veröffentlichungstag der Anmeldung:
**17.10.90 Patentblatt 90/42**

(84) Benannte Vertragsstaaten:
**AT BE CH DE DK ES FR GB GR IT LI LU NL SE**

(71) Anmelder: **Dr. Karl Thomae GmbH**
**Postfach 1755**
**D-7950 Biberach 1(DE)**

(72) Erfinder: **Werner, Rolf-Günter, Dr.**
**Hugo-Häring-Strasse 72**
**D-7950 Biberach 1(DE)**
Erfinder: **Berthold, Wolfgang, Dr.**
**Friedrich-Ebert-Strasse 32**
**D-7950 Biberach 1(DE)**
Erfinder: **Werz, William, Dr.**
**Am Espach 5**
**D-7951 Warthausen(DE)**
Erfinder: **Jung, Günther, Prof. Dr.**
**Ob der Grafenhalde 5**
**D-7400 Tübingen(DE)**
Erfinder: **Gombert, Frank Otto**
**Knollstrasse 4**
**D-7400 Tübingen-Bühl(DE)**

(54) **Monoklonale Antikörper gegen humane Gewebeplasminogenaktivatoren mit definierter Epitopspezifität, deren Herstellung und deren Verwendung.**

(57) Die hier vorliegende Erfindung betrifft monoklonale Antikörper gegen rekombinanten humanen Gewebeplasminogenaktivator mit definierter Epitopspezifität, deren Herstellung und deren Verwendung.

EP 0 392 369 A1

## Monoklonale Antikörper gegen humane Gewebeplasminogenaktivatoren mit definierter Epitopspezifität, deren Herstellung und deren Verwendung

In der Literatur werden bereits monoklonale Antikörper gegen verschiedene Gewebeplasminogenaktivatoren (t-PA) beschrieben, wobei bei der Herstellung der literaturbekannten monoklonalen Antikörpern das Gesamtmolekül t-PA als Immunogen verwendet wird. Diese Antikörper weisen daher den Nachteil auf, daß deren Epitopspezifitäten nicht bekannt sind, da bei den beschriebenen Immunisierungen das Immunsystem des Wirts theoretisch ein weites Spektrum von Antikörpern gegenüber allen erkennbaren Stellen auf dieser artfremden Substanz produzieren kann, vorausgesetzt, daß diese Substanz oder Teilbereiche davon für den betreffenden Organismus immunogen ist.

t-PA ist ein sogenanntes konservatives Molekül, das eine große Aminosäuresequenzhomologie innerhalb der verschiedenen Spezies aufweist. Die nach einer Immunisierung und anschließender Fusion zu erwartenden monoklonalen Antikörper, die sich in ihrer Epitopspezifität voneinander unterscheiden, sind daher limitiert. Dies bedeutet, daß der betreffende Organismus, der als Antikörperproduzent dient, den überwiegenden Teil des hu rt-PA's oder t-PA's als artfremd nicht erkennen kann, da das Gesamtmolekül t-PA fast identisch oder sehr eng verwandt zu seinem körpereigenen t-PA ist. Folglich kann dieser Organismus nur sehr wenige voneinander verschiedene Antikörper bilden, die in ihrer Epitopspezifität different sind.

Die Anzahl dieser voneinander verschiedenen Antikörper können qualitativ noch weiter limitiert sein, da bei einer Immunisierung mit dem Gesamtmolekül immunogen dominante Stellen die Immunantwort beeinflussen und somit bei einer Fusion nur monoklonale Antikörper gegen diese immunogen dominanten Stellen gewonnen werden können. Weniger immun-dominante Epitope des t-PA Moleküls werden somit bei einer Fusion nicht erfaßt.

Dies bedeutet, daß von den bereits bekannten monoklonalen Antikörpern gegen hu rt-PA nicht alle vorhandenen Epitope erkannt werden sondern nur einige wenige, wobei es zudem völlig unklar ist, welches Epitop von welchem Antikörper erkannt wird. Dabei ist außerdem noch zu unterscheiden, ob es sich um ein Sequenzepitop oder ein Strukturepitop handelt. Handelt es sich um ein Sequenzepitop oder kontinuierliches Epitop, so erkennt der monoklonale Antikörper die mehr oder weniger lineare Form einer gegebenen Aminosäuresequenz. Bei einem Strukturepitop oder diskontinuierlichen Epitop erkennt der monoklonale Antikörper die räumliche Anordnung eines oder mehrer Teilpeptide. Die Aminosäuresequenz eines Epitopes muß also nicht zwangsläufig eine linearisierte Aminosäuresequenz (Primär- bzw. Sekundärstruktur) des Proteins darstellen, sondern ein Epitop kann auch durch die geordnete räumliche Faltung von Aminosäuresequenzen gebildet werden.

Die Epitopspezifität der literaturbekannten monoklonalen Antikörper für hu rt-PA kann nur unter erheblichem Aufwand mittels Peptidfragmenten, die entweder synthetisch oder enzymatisch hergestellt wurden, oder mittels Epitop-Scanning [siehe Geysen, H. M., Meloen, R. H. and Barteling, S. J. in Proc. Natl. Acad. Sci USA. 81, 3998 (1984); Geysen, H. M., Barteling, S. J. and Meloen, R. H. in Proc. Natl. Acat. Sci. USA 82, 178 (1985); The Peptides - Analysis, Synthesis and Biology, Eds. Udenfriend and Meienhofer, Volume 9, Academic Press (1987); Geysen, H. M., Rodda. S. J., Mason, T. J., Tribbick, G., and Schoofs, P. G. in J. Immunological Methods 102, 259 (1987); Geysen, H. M., Rodda. S. J. and Mason, T. J. in Mol. Immunol. 23, 709 (1986)] ermittelt werden.

Der vorliegenden Erfindung lag somit die Aufgabe zugrunde, neue monoklonale Antikörper gegen Gewebeplasminogenaktivatoren, insbesondere gegen rekombinaten humanen Gewebeplasminogenaktivator (hu rt-PA) mit definierter Sequenz-Epitopspezifität herzustellen, die jeweils ein vorbestimmtes der theoretisch möglichen Epitope, also auch die schwach immunogenen Epitope erkennen. Derartige Antikörper sind von großer Bedeutung, da es mit ihrer Hilfe möglich sein sollte

1. Vorhersagen über das Vorliegen von primären, sekundären und tertiären Strukturen von hu rt PA zu treffen,

2. t-PA von anderen Plasminogenaktivatoren zu unterscheiden, z.B. im Serum und in Zellkulturüberständen,

3. die verschiedenen Formen des t-PA's voneinander zu unterscheiden, z.B. die einkettige von der zweikettigen Form, TYP I von Typ II usw., und

4. Struktur-Funktions-Beziehungen verschiedener Bereiche des t-PA's wie z.B. der Fibronectin-Finger-Domäne, Wachstumsfaktor-Domäne, 1. und 2. Kringel-Domäne aufzustellen und gezielt zu untersuchen, und aus den gewonnenen Erkenntnissen die physiologischen Eigenschaften des t-PA's ganz gezielt zu verändern, insbesondere zu verstärken bzw. zu verbessern, z.B. durch Blockierung der Inhibitor-Bindungsstelle, durch Maskierung der Kohlenhydratanteile oder durch Maskierung der Rezeptorbindungsstelle für die rt-PA Elimination.

Erfindungsgemäß wurde diese Aufgabe wie folgt gelöst:

An Hand der Primärstruktur des t-PA's (siehe Pennica, D. et al. in Nature 301, 214-221) wurde mit Hilfe eines Computer-programmes postuliert, daß die Peptidsequenz von hu rt-PA 36 Epitope aufweist. Dabei verbindet die Computeranalyse die Vorhersagen der Parameter Hydrophilie (Hopp, T.P. and Woods, K.R. in Molec. Immun. 20, 483-489 (1983)), Hydropathie (Kyte, J. and Doolittle, F.R. in J. Mol. Biol. 157, 105-132 (1982)), Acrophilie (Hopp, T.P. in Annali sclavo 2, 47-60 (1984)), Antigenität und Flexibilität (Karplus, P.A. and Schulz, G.E. in Naturwissenschaften 72, 212-213 (1985)) mit den Vorhersagen über die Komformationsmerkmale ($\alpha$-Helix, $\beta$-turn, random coil) (Granier, J. et al. in J. Mol. Biol. 120, 97-120 (1978)) der zugrunde liegenden Aminosäuresequenzen. Die Kernbereiche der postulierten Epitope werden durch folgende Teilsequenzen der von Pennica, D. et al. in Nature 301, 214-221 veröffentlichten Aminosäuresequenz des t-PA's gebildet:

1-2 (1), 7-9 (2), 20-22 (3), 24-30 (4), 37-40 (5), 49-53 (6), 58-61 (7), 84-85 (8), 89-100 (9), 103 (10), 107-110 (11), 125-130 (12), 142-151 (13), 160-164 (14), 171-177 (15), 183-188 (16), 194-196 (17), 217-219 (18), 226-239 (19), 241-242 (20), 245-250 (21), 268-271 (22), 297-302 (23), 326-329 (24), 344-350 (25), 362-369 (26), 378-387 (27), 411-417 (28), 426-427 (29), 436-442 (30), 445-447 (31), 460-466 (32), 475-479 (33), 502-506 (34), 515 (35) und 522-527 (36).

Jedes einzelne dieser Epitopbereiche sollte daher immunogene Eigenschaften zeigen und somit als Antigen zur Bildung von monoklonalen Antikörpern geeignet sein. Dabei müssen die Kernbereiche so ergänzt werden, daß sie eine antigene Determinante darstellen. Hierzu werden die Kernbereiche analog der natürlichen Aminosäuresequenz des hu rt-PA auf 10 bis 15 Aminosäuren umfassenden Peptide ergänzt.

Erfindungsgemäß erhält man die neuen monoklonalen Antikörper nach einem bekannten Verfahren, das von Köhler und Milstein erstmals in Nature 256, 495-497 (1975) beschrieben wurde.

Dieses Verfahren besteht grundsätzlich darin, daß man einer Maus oder einem anderen geeigneten Empfänger ein geeignetes Immunogen injiziert. Nach erfolgreicher Immunisierung wird die Milz entnommen, in Einzelzellen dissoziiert und mit Myelomazellen fusioniert. Man erhält Hybridzellen, die als Hybridoma bezeichnet werden. Diese Hybridomazellen reproduzieren sich in-vitro. Durch die Wahl der Selektionsbedingungen und durch Klonierung (limiting dilution) werden einzelne Klone isoliert, von denen jeder eine einzige epitopspezifische Antikörperspezies gegenüber dem Antigen synthetisiert. Jede auf diese Weise erhaltene individuelle Antikörperspezies ist das Produkt einer einzigen fusionierten B-Zelle aus dem entsprechend immunisierten Organismus, die erzeugt wurde als Reaktion auf eine antigene Determinante der immunogenen Substanz (siehe auch Kearney J. et al in J. Immunol. 123, 1548-1558 (1979); Galfre and Milstein in Meth. Enzym. 73, 3-46 (1981) und Yelter in Somatic Cell Genetic 3, 231-242), (1977)).

Erfindungsgemäß wird also bei dem hier beschriebenen Verfahren als Epitop mindestens eines der vorstehend erwähnten und als Epitop klassifizierten Peptid-Fragmente (1) bis (36), welches an einen Adjuvant-Carrier wie z. B. an Hämocyanin, an Katalase oder an ein Lipoprotein, vorzugsweise jedoch an Katalase, gekoppelt ist, oder ein Derivat hiervon verwendet, wobei zweckmäßigerweise die ermittelten Sequenzbereiche so ergänzt werden, daß sich peptidsynthetisch sinnvolle Segmente, die mindestens 10 bis 15 Aminosäuren umfassen, ergeben.

Die Immunisierung kann auch mittels enzymatischer Spaltfragmente des rekombinant hergestellten hu rt-PA's unterschiedlicher Länge, mittels entsprechend definierter, chemisch analog der natürlichen Aminosäuresequenz synthetisierter Peptide oder mittels möglicher Varianten des hu rt-PA, erfolgen. Dabei können ein oder mehrere unterschiedlich definierte Epitope an den gleichen Carrier gekoppelt sein. Es können also die eingesetzten Immunogene, abgeleitet von hu rt-PA, untereinander oder sequenziell beliebig variiert werden, z. B. kann die 1. Immunisierung mit $(XYZ)_m$ (rt-PA) gekoppelt an Katalase oder ungekoppelt, die 2. Immunisierung mit $(XYZ)_m$ rt-PA gekoppelt an Pam$_3$ Cys oder ungekoppelt usw. erfolgen, wobei $(XYZ)_m$ rt-PA für jede beliebige Aminosäuresequenz des hu rt-PA Moleküls oder dessen Varianten stehen kann und nicht auf ein Tripeptid oder vielfaches davon beschränkt ist. Die Immunisierung kann jedoch auch in vitro erfolgen.

Auf Grund des obigen Postulats wurde beispielsweise das hu rt-PA 343-353 (25)-Fragment, welches das 344-350 (25)-Fragment enthält, mittels üblicher Methoden synthetisiert. Am Beispiel dieses Fragmentes wurde das erfindungsgemäße Verfahren vorzugsweise wie folgt durchgeführt:

1. Zur Immunisierung werden Balb/c-Mäuse im 2-Wochenrhythmus wie folgt immunisiert:

Das hu rt-PA 343-353 (25)-Fragment, welches vorzugsweise an Katalase gekoppelt ist, wird vorzugsweise in 250 $\mu$l PBS plus 250 $\mu$l komplettes Freund'sches Adjuvant appliziert, wobei erforderlichenfalls für weitere Immunisierungen, vorzugsweise für die 2. bis 6. Immunisierung, anstatt komplettes inkomplettes Freund'sches Adjuvant verwendet wird.

2. Die Fusion, Selektion, Produktion usw. von monoklonalen Antikörpern erfolgt ebenfalls nach bekannten Methoden (siehe Köhler and Milstein in Nature 256, 495-497 (1975); Kearney J. et al in J. Immunol. 123, 1548-1558 (1979); Galfre and Milstein in Meth. Enzym. 73, 3-46 (1981) und Yelter in Somatic Cell Genetic 3, 231-242), (1977)).

Alle Klone die eine positive Reaktion gegen das Immunogen und gegenüber hu rt-PA zeigen wurden 4 Wochen auf ihre stabile Antikörperproduktion getestet. Außerdem wurde ihre Kreuzreaktivität gegenüber anderen Proteinen, wie z.B. Urokinase, Trypsin, Chymotrypsin, Elastase, Streptokinase, Prourokinase, Katalase, Serumalbumin, Epidermal Growth Factor und Fibronectin, getestet (siehe Tabelle 1). Nur solche Klone, die keine Kreuzreaktivität gegenüber den aufgezeigten Proteinen zeigten, wurden für eine Produktion verwendet.

Die Produktion der monoklonalen Antikörper kann in verschiedenen Volumina erfolgen, z.B. in Gewebekulturschalen, Gewebekulturflaschen, Spinnergefäße oder Fermentern. Dabei können je nach Anforderungen verschiedene Produktionsmedien Verwendung finden, wie z.B. (RPMI + ≧ 10 % FCS) oder geeignete definierte serumfreie Medien.

Die Epitopspezifität der monoklonalen Antikörper (MAK) wird mittels Peptid-Scanning [siehe Geysen, H. M., Meloen, R. H. and Barteling, S. J. in Proc. Natl. Acad. Sci USA. 81, 3998 (1984); Geysen, H. M., Barteling, S. J. and Meloen, R. H. in Proc. Natl. Acat. Sci. USA 82, 178 (1985); The Peptides -Analysis, Synthesis and Biology, Eds. Udenfriend and Meienhofer, Volume 9, Academic Press (1987); Geysen, H. M., Rodda. S. J., Mason, T. J., Tribbick, G., and Schoofs, P. G. in J. Immunological Methods 102, 259 (1987); Geysen, H. M., Rodda. S. J. and Mason, T. J. in Mol. Immunol. 23, 709 (1986)] ermittelt. Das Antikörper-Peptid-Scanning (Pepscan) wurde beispielsweise wie folgt durchgeführt:

Überlappende 8-Aminosäuren lange Peptide des t-PA's wurden wie oben beschrieben synthetisiert. Diese Methode erforderte die Synthese von 520 überlappenden Peptiden der 527 Aminosäuren des t-PA's. Peptid 1 besteht aus den Aminosäuren 1-8, Peptid 2 aus den Aminosäuren 2-9, und so weiter. Die Peptide bleiben nach der Synthese an dem festen Träger gebunden. Hybridomzellkulturüberstände oder deren gereinigte monoklonalen Antikörper werden in ELISA-Platten in geeigneten Verdünnungen auf ihre Reaktivität gegenüber diesen Peptiden getestet.

Darüber hinaus kann mittels weiterer Peptidfragmenten, die entweder ebenfalls synthetisiert oder enzymatisch hergestellt wurden, das Epitop der monoklonalen Antikörper weiter analysiert werden. Durch weitere Analyse der Reaktivität gegenüber der nativen Form des t-PA's oder dessen reduzierter carboxymethylierter Form ist die Aussage möglich, ob der monoklonale Antikörper ein Sequenzepitop (kontinuierliches Epitop), ein Strukturepitop (diskontinuierliches Epitop) oder beide Epitopformen erkennen kann. Wenn das Sequenzepitop in seiner linearisierten Form gleichzeitig ein Strukturepitop bildet, das räumlich am Molekül exponiert ist, so liegt ein kontinuierliches Epitop vor. Dann reagiert der monoklonale Antikörper mit dem nativen hu rt-PA und dessen reduziertercarboxymethylierter Form. Reagiert der monoklonale Antikörper aber nur mit der nativen, dreidimensionalen Struktur des Moleküls und nicht mit dessen linearisierter Form, also mit dessen reduzierter-carboxymethylierter Form, so liegt ein diskontinuierliches Epitop vor. Reagiert der monoklonale Antikörper nur mit der reduzierter-carboxymethylierten Form und nicht mit der nativen Form, so kann ebenfalls ein diskontinuierliches Epitop vorkommen oder das kontinuierliche Epitop liegt nicht an der Oberfläche oder ist aus sterischen Ursachen nicht für den Antikörper zugänglich. Wenn der monoklonale Antikörper mit der nativen Form und mit mehreren unterschiedlichen Sequenzbereichen des reduzierten-carboxymethylierten hu rt-PA reagiert, so kann ein diskontinuierliches Epitop vorliegen. Die linearen Sequenzbereiche sind dann durch die räumliche Faltung in enge Nachbarschaft gekommen.

Da die monoklonalen Antikörper 17-134/11 und 17-124/20 hu rt-PA in seiner nativen und reduziertercarboxymethylierten Form erkennen (siehe Tabelle 1), ist deren Epitop auf der Oberfläche des nativen rt-PA exponiert und stellt auch bei der tertiären Konformationsausbildung ein kontinuierliches Epitop mit der essentiellen Aminosäuresequenz EEEQK dar (siehe Tabellen 2 und 3). Dieses Pentapeptid stellt die minimale Aminosäuresequenz dar, die der Antikörper für seine Bindung benötigt, N-terminal begünstigt die Sequenz VPG und C-terminal die Sequenz FEV die Bindung des Antikörpers an dessen Epitop (siehe Tabellen 2 und 3). Faßt man diese Ergebnisse zusammen, so bindet der monoklonale Antikörper 17-134/11 bzw. 17-134/20 an ein Epitop in dem Sequenzbereich VPGEEEQKFEV, in welchem das Fragment (25) mit der Aminosäuresequenz

$_{344}$Val-Pro-Gly-Glu-Glu-Glu-Gln-Lys-Phe-Glu-Val$_{354}$

enthalten ist.

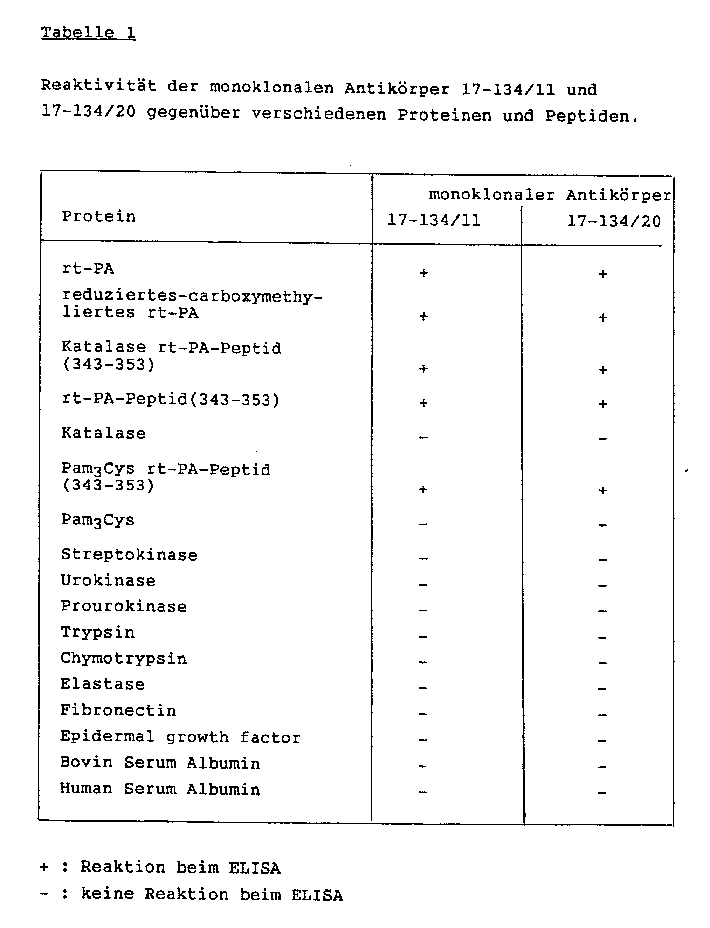

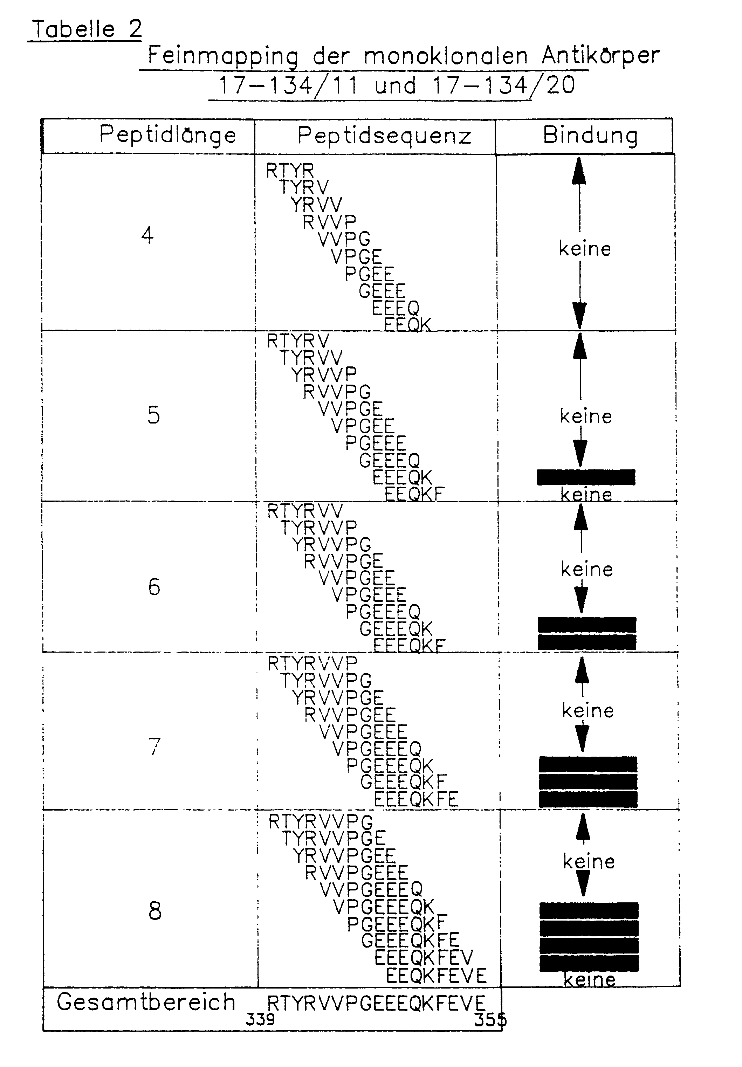

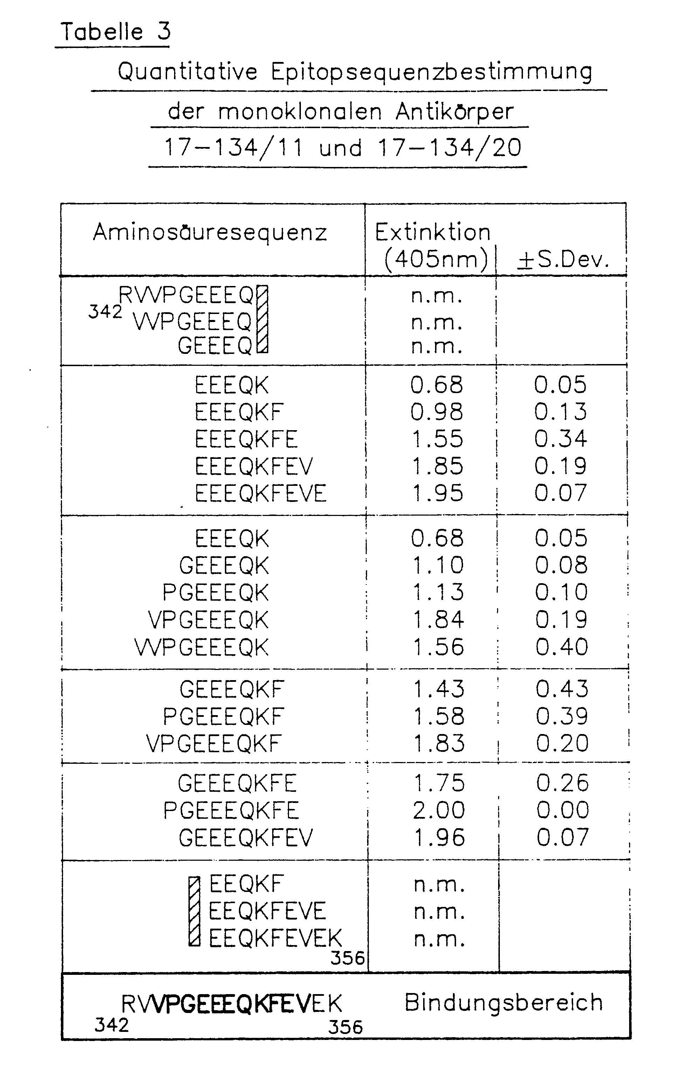

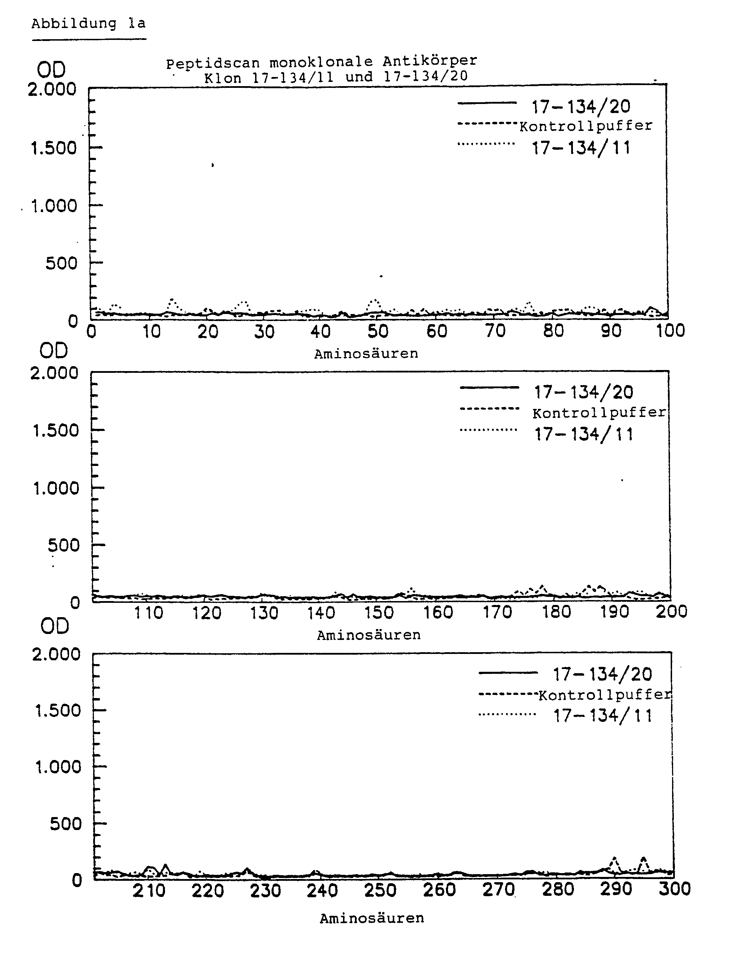

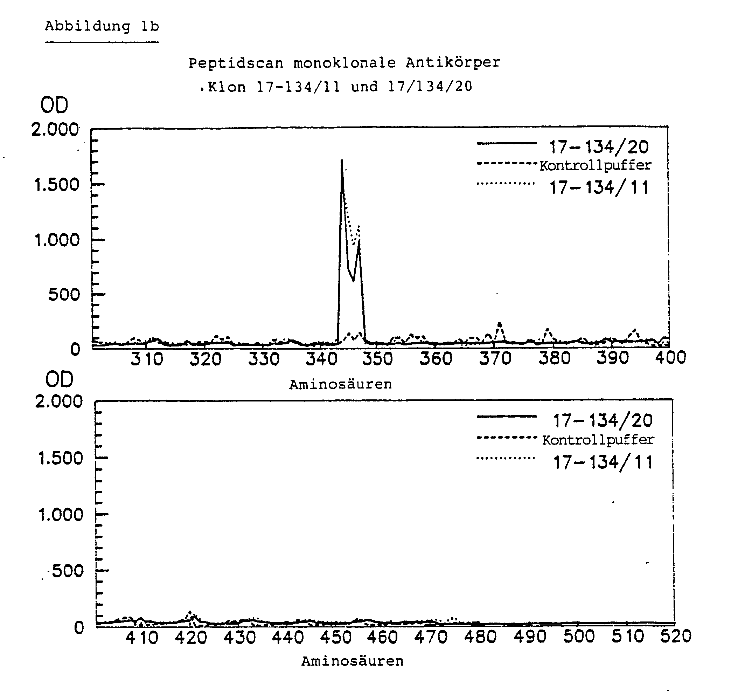

Die Tabellen 2 und 3 sowie die Abbildungen 1a und 1b zeigen die Analyse des MAK 17-134/20 bzw. 17-134/11 mittels Peptid-Scanning, die erfindungsgemäß durch eine gezielte Immunisierung mittels vorhergesagten Epitopsequenzen des hu rt-PA gewonnen wurden.

Die so hergestellten Antikörper der Klone 17-134/20 bzw. 17-134/11 gehören der Immunglobulinsubklasse IgM an. Trotz langfristiger Immunisierung, bei der hauptsächlich IgG-Antikörpersubklassen produzierende Klone erwartet werden, führte die Fusion zu IgM-Antikörpermolekülen. Diese Tatsache steht im Einklang mit den Beobachtungen, daß bei einer Immunisierung mit konservativen Molekülen, wie dem hu rt-PA überwiegend IgM-Antikörper gebildet werden, unabhängig von dem Immunisierungsschema. Es ist jedoch jederzeit möglich auf IgG-Antikörper oder anderen Ig-Subtypen zu screenen, die identische epitopspezifische Eigenschaften besitzen wie die beschriebenen monoklonalen IgM-Antikörper.

Die so gewonnenen und epitopcharakterisierten MAK können als Diagnostikum, zur Qualifizierung und Quantifizierung (z. B. ELISA usw.), zur Reinigung von hu rt-PA (z. B. Affinitätschromatographie), für Drug targeting (Kopplung an rt-PA), als Therapeutikum (Neutralisation von t-PA) oder Pharmazeutikum (z.B. Maskierung der Rezeptorbindungsstelle für die Elimination) eingesetzt werden. Dabei können die Antikörper in ihrer nativen Form oder als Fragmente Verwendung finden. Desweiteren können die Antikörper oder deren Fragmente entsprechend markiert oder modifiziert werden.

Sollen die neuen monoklonalen Antikörper beispielsweise zum Nachweis von hu rt-PA im Serum oder in Körperflüssigkeiten bestimmt werden, kommen hierbei insbesondere Immunoassaytechniken in Betracht. Diese Techniken basieren auf der Bildung eines Komplexes zwischen der zu bestimmenden antigenen Substanz und einem Antikörper oder Antikörpern, in denen ein oder mehrere Teile des Komplexes markiert sein können. Erfolgt diese am Antigen, z.B. durch ein radioaktives Marker-Isotop wie $^{125}$Jod, so wird vorzugsweise ein kompetiver Verdrängungsassay, z.B. der Radioimmunoassay (RIA), benutzt.

Erfolgt die Markierung am Antikörper, z.B. durch Radioaktivmarkierung, Fluoreszenzmarkierung, Kopplung an Markerenzymen oder Kopplung an Metallchelaten, so wird vorzugsweise ein Immuno-Radiometrischer Assay (IRMA) oder ein Enzyme Linked Immunosorbent-Assay (ELISA) benutzt.

Eine andere Möglichkeit, die auch zu einer höheren Empfindlichkeit des Nachweises führen kann, besteht in der Kopplung der Antikörper mit niedermolekularen Haptenen. Diese können dann ihrerseits durch eine zweite Reaktion spezifisch nachgewiesen werden. Gebräuchlich sind z.B. Biotin (reagierend mit Avidin) oder Dinitrophenyl, Pyridoxal, Fluoreszamin (reagierend mit jeweils spezifischen Antihapten-Antikörper).

Die exquisite Spezifität von monoklonalen Antikörpern erlaubt es den empfindlichen Assaytyp, d.h. die Markierung des Antikörpers zu wählen. Als Markierungssignal eignen sich besonders Enzyme, da die Anwendung von Radioisotopen mit großem Aufwand zum Schutz der Ausführenden und der Umwelt verbunden ist. Außerdem stellt die katalytische Aktivität eines Enzyms zudem eine Amplifikation des Signals dar. Besonders geeignet ist die Meerrettichperoxidase, da es eine Reihe von sehr guten Substraten gibt. Zudem kann das kleine Enzym mit einer einfachen, aber spezifischen Methode (Perjodatmethode) an Antikörper gebunden werden.

Wie bereits erwähnt, können die neuen monoklonalen Antikörper, die gegen den Proteasenteil des hu rt-PA gerichtet sind, die pharmazeutische Wirkung des hu rt-PA ebenfalls neutralisieren, oder

die neuen monoklonalen Antikörper, die gegen die Kohlenhydratanteile des hu rt-PA's gerichtet sind, den Abbau des hu rt-PA's in der Leber verzögern und somit die physiologische Halbwertszeit des hu rt-PA's verlängern oder

die neuen monoklonalen Antikörper, die mit der Inhibitorbindung kompetitieren, die Halbwertszeit von hu rt-PA verlängern.

Ferner kann hu rt-PA oder dessen Erscheinungsformen (Typ I, Typ II, Einketten-Zweiketten usw.) im Serum und anderen Flüssigkeiten von anderen Proteasen-aktivatoren diskriminiert und somit qualitativ und quantitativ erfaßt werden:

Durch Vergleich der Reaktivität der monoklonalen Antikörper gegenüber der nativen Form des hu rt-PA's und seiner reduzierten-carboxymethylierten Form werden Aussagen über die Sekundär- und Tertiärstruktur möglich.

Desweiteren ist es durch gezielten Aminosäurenaustausch der synthetisierten Peptide möglich, die Immunogenität nicht oder schwach immunogener Sequenzen zu verändern und dadurch das Spektrum der produzierbaren monoklonalen Antikörper gegen hu rt-PA zu erweitern. Dadurch kann man weitere Rückschlüsse auf die drei dimensionalen Struktur der durch Computeranalyse vorhergesagten exponierten, linearen Sequenzen des hu rt-PA erhalten. Dadurch kann man anschließend hu rt-PA derart in seiner Struktur verändern, um zu hu rt-PA Molekülen 2. Generation zu gelangen, die eine verbesserte Wirkungsweise, z.B. eine verlängerte Halbwertszeit und keine Bindungsaffinitäten für junge Fibringerüste, aufweisen und die, obwohl sie eine Variante des natürlichen hu rt-PA darstellen, keine oder verminderte immunogene Eigenschaften besitzen. Umgekehrt kann durch die Computervorhersage und durch spezifisch synthetisierte Peptide die Immunogenität vorhandener oder geplanter Varianten des hu rt PA (rt-PA 2. Generation) überprüft werden.

Die nachfolgenden Beispiele sollen die Erfindung näher erläutern, wobei die nachfolgenden Abkürzungen verwendet werden:

AA: Aminosäure

ABTS: 2,2'-Azino-bis(3-ethylbenzthiazoline-6-sulfonic acid)

Ac$_2$O: Acetanhydrid

Aminosäuren: A: Alanin (Ala), C: Cystein (Cys), D: Asparaginsäure (Asp), E: Glutaminsäure (Glu), F: Phenylalanin (Phe), G: Glycin (Gly), H: Histidin (His), I: Isoleucin (Ile), K: Lysin (Lys), L: Leucin (Leu), M: Methionin (Met), N: Asparagin (Asn), P: Prolin (Pro), Q: Glutamin (Gln), R: Arginin (Arg), S: Serin (Ser), T: Threonin (Thr), V: Valin (Val), W: Tryptophan (Trp) Y: Tyrosin (Tyr)

ASA: Aminosäureanalyse

BOC: tertiär-Butyloxycarbonyl

BOP: Benzotriazol-1-yl-oxy-tris-(dimethylamino)phosphoniumhexafluorophosphat

BSA: Bovine serum albumin

D: Dalton

DCC: Dicyclohexylcarbodiimid

DCM: Dichlormethan

DIC: Diisopropylcarbodiimid

DIPEA: N,N-Diisopropylethylamin

DMF: Dimethylformamid

EDC: N-(3-Dimethylaminopropyl)-N'-ethylcarbodiimid

ELISA: Enzyme Linked Immunosorbent Assay

Fmoc: 9-Fluorenylmethoxycabonyl

Fmoc-AS: Fluorenylmethoxycarbonyl-Aminosäure

HOBt: Hydroxybenzotriazol

hu: human

MAK: Monoklonaler Antikörper

MeOH: Methanol

MIT: Mikrotiterplatte

NMP: N-Methylpyrolidon

OtBu: tertiär-Butylester

OVA: Ovalbumin

PBS: Phosphate buffered saline

Pam$_3$Cys: N-Palmitoyl-S-[(2RS)-2,3-bis(palmitoyl)propyl]-Cystein

Pam$_3$C-OH: Tripalmitoyl-S-glyceryl-L-cystein

rt-PA: recombinanter tissue-type plasminogen activator

RT: Raumtemperatur

tBu: tertiär-Butyl

TFA: Trifluoressigsäure

V: Volumen


Beispiel 1


Synthese des Katalase-Konjugates

Die Festphasenpeptidsynthese des Peptides t-PA (343-354) NH$_2$-VVPGEEEQKFEV-OH erfolgte am vollautomatischen Peptidsynthesizer 430A (ABI, Weiterstadt, FRG). Die Fmoc-Aminosäuren Fmoc-V-OH, Fmoc-P-OH, Fmoc-G-OH, Fmoc-E(OtBu)-OH, Fmoc-Q-OH, Fmoc-K(Boc)-OH, Fmoc-F-OH, Fmoc-S(OtBu) sowie das Fmoc-V-para-Alkoxybenzylalkohol 1 % vernetztes Polystyrol-Divinylbenzol-Harz (Beladung: 0,43 mmol Fmoc-V/g Harz) wurden von Novabiochem AG, Läufelfingen, Schweiz bezogen. Nach Abspaltung der Fmoc-Schutzgruppe mittels Piperidin wird jede Aminosäure zweifach gekuppelt. Zunächst als Aktivester mittels DIC/HOBt in DMF und anschließend als symmetrisches Anhydrid mittels DIC in NMP. Fmoc-Q-OH wurde zweimal in NMP als symmetrisches Anhydrid nach verkürzter Voraktivierung gekuppelt.

Die Synthese erfolgte mit 500 mg (0.215 mmol) Fmoc-V-Harz. Nach Anbau der N-terminalen Aminosäuren Fmoc-V-OH wird das Peptidharz geteilt. Eine Hälfte wird zur Synthese des Katalase-Peptid-Konjugates eingesetzt, die andere Hälfte zur Ankupplung von Fmoc-S(tBu)-OH zur Synthese des Pam$_3$Cys-Ser-

Konjugates eingesetzt. Die Abspaltung der N-terminalen Fmoc-Schutzgruppe erfolgt durch 15-20 minütiges Schütteln des Peptidharzes in 20 % Piperidin/DMF in einer Schüttelente. Das Harz wird anschließend 2 x mit DMF, 2 x mit DCM, 2 x mit MeOH und 2 x mit DCM gewaschen und am Wasserstrahlvakuum getrocknet.

Die Abspaltung des Peptides vom Harz erfolgt mit TFA/Resorcin/Thioanisol (95/2, 5/2,5; v/v/v) 2 Stunden bei RT unter Schütteln. Die Harzsuspension wird durch eine Glasfritte (G2) in einen Spitzkolben filtriert und das Harz zweimal mit TFA gewaschen. Die TFA-Lösung mit Peptid wird im Vakuum ein geengt, in möglichst wenig Essigsäure aufgenommen, und von den enthaltenen Scavengern (Resorcin und Thioanisol) und abgespaltenen Schutzgruppen durch mehrmaliges Ausfällen in kalten Diethylether, Durchmischen und Zentrifugieren des ausgefallenen Peptides gereinigt. Den Ether läßt man abdampfen und lyophilisiert zweimal aus tert.-Butanol/$H_2O$ (4/1; v/v).

Ausbeute: 160,3 mg Rohpeptid

Hydrolysebedingungen: 18 Stunden, 6N HCl 0,01 % Phenol, 100°C

ASA: E 5,8 (5), P 1,0 (1), G 1,0 (1), V 2,1 (3), F 1,2 (1), K 1,1 (1) (Angaben in Klammer: theoretisch berechnete Werte)

Prozentualer D-Anteil: E 2,0 %, P 0 %, V 0 %, F 0 %, K 3 %

Hydrolysebedingungen: 30 min, TFA/HCl (2/1; v/v), 165°C

ASA: E 5,6 (5), P nicht bestimmt, G 1,0 (1), V 2,5 (3), F 1,1 (1), K 1,0 (1)

Die Kupplung des Peptides an die Katalase (Aspergillus niger; Serva, Heidelberg, FRG) erfolgte in wäßriger Lösung mit einem 30-fachen Überschuß an wasserlöslichem Carbodiimid (EDC) und Peptid (Reaktionsdauer: 18 Stunden). Entstandener Harnstoff und nicht umgesetztes Carbodiimid und Peptid wurden durch Dialyse (Ausschlußgrenze: 3000 D) gegen Wasser (15 Stunden, dreimaliger Wasserwechsel) entfernt. Das Reaktionsprodukt wurde zweimal lyophilisiert, zunächst aus Wasser, dann aus tert.-Butanol/Wasser (4/1; v/v). Die Belegung der Katalase mit Peptid wurde mittels Aminosäureanalyse nach der Hydrolyse (6 N Salzsäure, 110°C/18 Stunden) von Katalase und Katalsekonjugat mit 20-25 Peptidmolekülen/Katalasemolekül ermittelt.

## Beispiel 2

### Fusion der Zellen

Die Milz einer immunisierten Maus wird unter sterilen Bedin gungen entnommen und die Milzzellen mit RMPI 1640 (Gibco, Karlsruhe) mit Hilfe einer Spritze aus dem Organ ausgespült. Die isolierten Milzzellen werden pelletiert (10 Minuten bei 1100 rpm), dreimal gewaschen (RPMI) und in RPMI 1640 aufgenommen. Die so gewonnenen Milzzellen werden mit P3X63Ag8.653 (ATCC CPL 1580) Myelomzellen fusioniert. Dazu werden die kultivierten Myelomzellen, die sich in der log-Phase befinden, ebenfalls pelletiert und dreimal gewaschen. 2 x $10^7$ Milzzellen und 2 x $10^7$ Myelomzellen werden in ein Falconröhrchen pipettiert, abzentrifugiert und zu dem Zellpellet 0,6 ml 40%iges Polyethylenglykol 4000 (Merk, Darmstadt) tropfenweise zugegeben (1 Minute). Innerhalb der folgenden 5 Minuten wird der Fusionsansatz mit ca. 10 ml RPMI und 10 ml HAT-Medium (Boehringer Mannheim) verdünnt, die Zellen repelletiert und in 25 ml HAT-Medium resuspendiert. Je 250 µl Zellsuspension werden pro well einer 24-well-cluster-Platte (Tecnomara, Fernwald) ausgesät und in einem $CO_2$-Inkubator inkubiert. Als Feederzellen dienen Peritonealmakrophagen, die bereits 1 Tag vorher in Kultur genommen werden (ca. 4 x $10^4$ Zellen pro well in HAT-Medium). Alle 3-5 Tage wird frisches HAT-Medium zugegeben. Je nach Wachstum der Fusionszellen werden nach ca. 2 Wochen Zellkulturüberstände abgenommen und mittels ELISA auf ihre Reaktivität getestet.

## Beispiel 3

### Screening der Hybridomzellen

Insgesamt werden 56 Klone auf ihre Reaktivität in ELISA (Enzyma Linked Immunosorbent Assay) getestet. Als Immunosorbent fand das Immunogen (Peptid 343-354), rt-PA, reduziertescarboxymethyliertes rt-PA und Katalase Verwendung (jeweils 2 µg/ml). Dabei zeigten die Klone mit der Bezeichnung 17-134/11 und 17-134/20 eine eindeutige Reaktion gegenüber dem Immunogen (Peptid 343-354), rt-PA und reduziertem-carboxymethyliertem rt-PA. Gegen Katalase konnte keine Reaktivität gemessen werden.

Durchführung des ELISA-Testes:

1. Beschichtung der Mikrotiterplatten (MIT) mit 100 µl Antigenlösung/well bei 4° C über Nacht;

2. Waschen der Mikrotiterplatten mit PBS/Tween 20 (0,15 %) 3-4 mal;

3. Blockierung der Mikrotiterplatten mit 1-1,5%iger BSA/PBS Lösung (pH 7.4): 200µl/well über 2 Stunden bei Raumtemperatur;

4. Waschen der Mikrotiterplatten (siehe oben);

5. Auftragung der Antikörper Proben in 0,5%iger BSA/PBS-Lösung, 100 µl/well; 2 Stunden bei Raumtemperatur stehen lassen;

6. Waschen der Mikrotiterplatten: 5 mal (siehe oben);

7. Auftragung des Anti-Ig-Antikörpers gekoppelt an Peroxidase (Rabbit-anti-Mouse-Peroxidase) in 0,5%iger BSA Lösung (1:20 000), 100 µl/well, 2 Stunden bei Raumtemperatur stehen lassen;

8. Waschen der Mikrotiterplatten: 5 mal (siehe oben);

9. Auftragung der ABTS-Lösung (100 mg ABTS, 50 mg Natriumperborat in 100 ml 0,1M Citratpuffer pH 4.2) mit 100 µl/well;

10. Messung bei 405 nm nach einer Reaktionszeit von 30 Minuten (bei Raumtemperatur) mit Hilfe des Microplate Readers (Dynatech MR 600).

Beispiel 4

Epitop-Charakterisierung der monoklonalen Antikörper 17-134/11 und 17-134/20 in Peptidscan

Die Identifikation der Bindungsstelle der monoklonalen Antikörper gegen rt-PA erfolgt mit Hilfe der Methode des Peptid-Scannings.

Die gesamte Aminosäuresequenz des rt-PA Moleküls wird in sich überlappende Fragmente von 8 Aminosäuren Länge (1-8, 2-9, 3-10 usw.) geteilt und diese Oktapeptide durch Fmoc-Festphasen-Synthese an die Spitze der Polyethylen-Stäbchen synthetisiert. Die Stäbchen (Pins) sind in einer Halterung befestigt, die in Bezug auf Format und räumliche Abmessung einer 96 well Mikrotiterplatte entsprechen:

|   | 1 | 2 | 3 | 4 | . |
|---|---|---|---|---|---|
| A | 1-8 | 2-9 | 3-10 | 4-11 | . |
| B | 13-20 | 14-21 |   |   |   |
| . |   |   |   |   |   |
| . |   |   |   |   |   |
| . |   |   |   |   |   |

Zudem werden Tetra-, Penta-, Hexa-, Hepta- , Okta- und Nonapeptide synthetisiert, um das Epitop exakt eingrenzen zu können.

Die Synthese der Peptide erfolgte an Polyethylen-Stäbchen (CRB, Cambridge, England) (Durchmesser: 4 mm, Länge 40 mm), die in einer 8 x 12 Matrix auf einem Halter des Formats einer Microtiter befestigt sind.

Zwischen Polyethylenpolymer und Peptid befindet sich folgender säure- und basenstabile Anker und Spacer aus Acrylsäure, Hexamethylendiamin und Fmoc-ß-Alanin:

Polyethylen-$CH_2CH_2$-CO-NH-$(CH_2)_6$-NH-CO-$CH_2$-NH-Fmoc

Die Kupplung der Aminosäuren erfolgt in Polyethylen-Microtiterplatten, alle Waschvorgänge werden in Polyethylen-Behältern durchgeführt. Die Kupplung der 8 bzw. 9 Aminosäuren erfolgt nach folgendem Schema:

| Lösungsmittel | Menge | Dauer | Funktion |
|---|---|---|---|
| 20 % Piperidin/DMF | 1,2 l | 30 min | Abspaltung der Fmoc-Schutzgruppe |
| 1 x DMF | 1,2 l | 5 min | waschen |
| 4 x Methanol | 1,2 | 2 min | waschen |
| | l | 10 min | trocknen |
| 1 x DMF | 1,2 l | 5 min | quellen |
| 3 $\mu$mol Fmoc-AS/ 3 $\mu$mol BOP 3 $\mu$mol HOBt/ 3,9 $\mu$mol DIPEA | 100 $\mu$l pro Loch | 60 min | Kupplung der Aminosäure |
| 1 x DMF | 1,2 l | 2 min | waschen |
| 4 x Methanol | 1,2 l | 2 min | waschen |
| 1 x DMF | 1,2 l | 2 min | waschen |

Die Abspaltung der letzten Fmoc-Schutzgruppe, Acetylierung des freien N-Terminus und Abspaltung der Aminosäureseitenketten-Schutzgruppen erfolgt nach folgendem Schema:

| Lösungsmittel | Menge | Dauer | Funktion |
|---|---|---|---|
| 20 % Piperidin/DMF | 1,2 l | 30 min | Abspaltung der Fmoc-Schutzgruppe |
| 1 x DMF | 1,2 l | 5 min | waschen |
| 4 x Methanol | 1,2 | 2 min | waschen |
| | l | 10 min | trocknen |
| 1 x DMF | 1,2 l | 5 min | quellen |
| DMF/Ac$_2$O/DIPEA 50/5/1 (v/v/v) | 1,2 l | 90 min | Acetylierung des N-Terminus |
| 1 x DMF | 1,2 l | 2 min | waschen |
| 4 x Methanol | 1,2 | 2 min | waschen |
| | l | 15 min | trocknen |
| TFA/Phenol/Thioanisol 95/2,5/2,5 (v/w/v) | 100 $\mu$l pro Loch | 90 min | Abspalten der Schutzgruppe |
| 2 x DMF | 1,2 l | 5 min | waschen |
| 2 x DCM | 1,2 l | 2 min | waschen |
| 2 x 5 % DIPEA/DMF | 1,2 l | 5 min | Neutralisieren der TFA |
| 1 x DMF | 1,2 l | 5 min | waschen |
| 1 x DCM | 2,0 l | 30 min | waschen im Ultraschallbad |
| 1 x Methanol | 2,5 | 18 h | waschen |
| | l | 18 h | trocknen |

Die Halter mit den fertigen Peptiden werden im Vakuum im Exsikkator über Silikagel aufbewahrt, und können direkt für den Pepscan-Elisa verwendet werden.

Der Test der monoklonalen Antikörper verläuft im wesentlichen wie ein konventioneller ELISA. Die für den ELISA-Pepscan benötigten Lösungen wie Blockierungspuffer, Probe usw. werden in (unbeschichtete) Mikrotiterplatten pipettiert und die in ihrer Halterung befindlichen 96 Stäbchen in diese wells der MIT gestellt.

Durchführung:

1. Blockierung der Stäbchen mit 1 % BSA/OVA/0,1 % Tween 20 Lösung; 200 $\mu$l/well einer MIT, 60 Minuten unter Rütteln;

2. Umsetzung der Stäbchen in eine Mikrotiterplatte mit der Antikörper-Probe: 175 µl/well, 60 Minuten bei Raumtemperatur oder über Nacht bei 4 °C;

3. Waschen der Stäbchen in PBS/Tween 20 Lösung: 4 mal 10 Minuten;

4. Umsetzen der Stäbchen in eine Mikrotiterplatte mit Anti-Ig-Antikörper:

Rabbit-anti-Mouse-Peroxidase (Dianova, Hamburg); Verdünnung 1 : 10 000, 175 µl/well, in 1 % BSA/OVA/0,1 % Tween 20 Lösung verdünnt, über 60 Minuten bei Raumtemperatur (Rütteln);

5. Waschen der Stäbchen (siehe oben);

6. Umsetzen der Stäbchen in eine Mikrotiterplatte mit ABTS Lösung (100 mg ATBS, 60 µl 35 % $H_2O_2$ in 200 ml 0,1M Citratpuffer pH 4,0): 150 µl/well, 30 Minuten;

7. Messung der Mikrotiterplatten bei 405 nm.


**Ansprüche**

1. Monoklonale Antikörper produzierende Hybridomzellinien, wobei jeder monoklonale Antikörper ein vorbestimmtes Epitop von einem humanen Gewebeplasminoaktivator erkennt, dadurch gekennzeichnet, daß diese durch Zellfusion von mit einem Peptid immunisierten Milzzellen, wobei das Peptid jeweils eines der folgenden Epitope mit den Aminosäuren 1-2 (1), 7-9 (2), 20-22 (3), 24-30 (4), 37-40 (5), 49-53 (6), 58-61 (7), 84-85 (8), 89-100 (9), 103 (10), 107-110 (11), 125-130 (12), 142-151 (13), 160-164 (14), 171-177 (15), 183-188 (16), 194-196 (17), 217-219 (18), 226-239 (19), 241-242 (20), 245-250 (21), 268-271 (22), 297-302 (23), 326-329 (24), 344-350 (25), 362-369 (26), 378-387 (27), 411-417 (28), 426-427 (29), 436-442 (30), 445-447 (31), 460-466 (32), 475-479 (33), 502-506 (34), 515 (35) und 522-527 (36) der von Pennica, D. et al. in Nature 301, 214-221 veröffentlichten Aminosäuresequenz des t-PA's einschließt, mit Myelomzellen erhalten werden.

2. Hybridomzellinien gemäß Anspruch 1, dadurch gekennzeichnet, daß als Milzzellen die von Balb/c-Mäusen und als Myelomzellen die der Linie P3X$_{63}$Ag8.653 oder ein Subklon hiervon verwendet werden.

3. Hybridomzellinien gemäß Anspruch 1 oder 2, dadurch gekennzeichnet, daß diese einen monoklonalen Antikörper produzieren, der den Epitopbereich 344-354 von hu rt-PA erkennt.

4. Hybridomzellinien gemäß Anspruch 3, bezeichnet als 17-134/11 und gekennzeichnet durch die Bildung eines monoklonalen Antikörpers mit den Merkmalen gemäß den Tabellen 1 bis 3 und Abbildungen 1a und 1b.

5. Hybridomzellinien gemäß Anspruch 3, bezeichnet als 17-134/20 und gekennzeichnet durch die Bildung eines monoklonalen Antikörpers mit den Merkmalen gemäß den Tabellen 1 bis 3 und Abbildungen 1a und 1b.

6. Monoklonale Antikörper vom IgG-Typ, vom IgM-Typ und von anderen Ig-Subtypen, dadurch gekennzeichnet, daß diese eines oder mehrere Epitope im Bereich der Aminosäuren 1-2 (1), 7-9 (2), 20-22 (3), 24-30 (4), 37-40 (5), 49-53 (6), 58-61 (7), 84-85 (8), 89-100 (9), 103 (10), 107-110 (11), 125-130 (12), 142-151 (13), 160-164 (14), 171-177 (15), 183-188 (16), 194-196 (17), 217-219 (18), 226-239 (19), 241-242 (20), 245-250 (21), 268-271 (22), 297-302 (23), 326-329 (24), 344-350 (25), 362-369 (26), 378-387 (27), 411-417 (28), 426-427 (29), 436-442 (30), 445-447 (31), 460-466 (32), 475-479 (33), 502-506 (34), 515 (35) und 522-527 (36) des t-PA's erkennen.

7. Monoklonale Antikörper gemäß Anspruch 6, dadurch gekennzeichnet, daß diese von einer Hybridomzellinie nach mindestens einem der Ansprüche 1 bis 4 produziert werden.

8. Verfahren zur Herstellung einer Hybridomzellinie nach mindestens einem der Ansprüche 1 bis 5, dadurch gekennzeichnet, daß Milzzellen eines durch ein Epitop im Bereich der Aminosäuren 1-2 (1), 7-9 (2), 20-22 (3), 24-30 (4), 37-40 (5), 49-53 (6), 58-61 (7), 84-85 (8), 89-100 (9), 103 (10), 107-110 (11), 125-130 (12), 142-151 (13), 160-164 (14), 171-177 (15), 183-188 (16), 194-196 (17), 217-219 (18), 226-239 (19), 241-242 (20), 245-250 (21), 268-271 (22), 297-302 (23), 326-329 (24), 344-350 (25), 362-369 (26), 378-387 (27), 411-417 (28), 426-427 (29), 436-442 (30), 445-447 (31), 460-466 (32), 475-479 (33), 502-506 (34), 515 (35) und 522-527 (36) immunisierten Versuchstieres oder durch in vitro-Immunisierung mit Myelomzellen fusioniert werden.

9. Verfahren zur Herstellung der monoklonalen Antikörper gemäß Anspruch 6 oder 7, dadurch gekennzeichnet, daß eine Hybridomzellinie nach mindestens einem der Ansprüche 1 bis 5 kultiviert wird und die gebildeten Antikörper isoliert werden.

10. Verwendung der neuen Antikörper gemäß Anspruch 6 oder 7 zu analytischen Zwecken, als Diagnostikum, zur Reinigung von hu rt-PA, für Drug targeting oder als Therapeutikum.

<u>Tabelle 1</u>

Reaktivität der monoklonalen Antikörper 17-134/11 und
17-134/20 gegenüber verschiedenen Proteinen und Peptiden.

| Protein | monoklonaler Antikörper | |
| --- | --- | --- |
| | 17-134/11 | 17-134/20 |
| rt-PA | + | + |
| reduziertes-carboxymethyliertes rt-PA | + | + |
| Katalase rt-PA-Peptid (343-353) | + | + |
| rt-PA-Peptid(343-353) | + | + |
| Katalase | − | − |
| $Pam_3Cys$ rt-PA-Peptid (343-353) | + | + |
| $Pam_3Cys$ | − | − |
| Streptokinase | − | − |
| Urokinase | − | − |
| Prourokinase | − | − |
| Trypsin | − | − |
| Chymotrypsin | − | − |
| Elastase | − | − |
| Fibronectin | − | − |
| Epidermal growth factor | − | − |
| Bovin Serum Albumin | − | − |
| Human Serum Albumin | − | − |

+ : Reaktion beim ELISA

− : keine Reaktion beim ELISA

## Tabelle 2

### Feinmapping der monoklonalen Antikörper 17-134/11 und 17-134/20

| Peptidlänge | Peptidsequenz | Bindung |
|---|---|---|
| 4 | RTYR<br>TYRV<br>YRVV<br>RVVP<br>VVPG<br>VPGE<br>PGEE<br>GEEE<br>EEEQ<br>EEQK | ↕ keine |
| 5 | RTYRV<br>TYRVV<br>YRVVP<br>RVVPG<br>VVPGE<br>VPGEE<br>PGEEE<br>GEEEQ<br>EEEQK<br>EEQKF | ↕ keine<br>▬ keine |
| 6 | RTYRVV<br>TYRVVP<br>YRVVPG<br>RVVPGE<br>VVPGEE<br>VPGEEE<br>PGEEEQ<br>GEEEQK<br>EEEQKF | ↕ keine<br>▬▬ |
| 7 | RTYRVVP<br>TYRVVPG<br>YRVVPGE<br>RVVPGEE<br>VVPGEEE<br>VPGEEEQ<br>PGEEEQK<br>GEEEQKF<br>EEEQKFE | ↕ keine<br>▬▬▬ |
| 8 | RTYRVVPG<br>TYRVVPGE<br>YRVVPGEE<br>RVVPGEEE<br>VVPGEEEQ<br>VPGEEEQK<br>PGEEEQKF<br>GEEEQKFE<br>EEEQKFEV<br>EEQKFEVE | ↕ keine<br>▬▬▬▬ keine |
| Gesamtbereich | RTYRVVPGEEEQKFEVE<br>339       355 | |

## Tabelle 3

Quantitative Epitopsequenzbestimmung

der monoklonalen Antikörper

17—134/11 und 17—134/20

| Aminosäuresequenz | Extinktion (405nm) | ±S.Dev. |
|---|---|---|
| 342 RVVPGEEEQ | n.m. | |
| VVPGEEEQ | n.m. | |
| GEEEQ | n.m. | |
| EEEQK | 0.68 | 0.05 |
| EEEQKF | 0.98 | 0.13 |
| EEEQKFE | 1.55 | 0.34 |
| EEEQKFEV | 1.85 | 0.19 |
| EEEQKFEVE | 1.95 | 0.07 |
| EEEQK | 0.68 | 0.05 |
| GEEEQK | 1.10 | 0.08 |
| PGEEEQK | 1.13 | 0.10 |
| VPGEEEQK | 1.84 | 0.19 |
| VVPGEEEQK | 1.56 | 0.40 |
| GEEEQKF | 1.43 | 0.43 |
| PGEEEQKF | 1.58 | 0.39 |
| VPGEEEQKF | 1.83 | 0.20 |
| GEEEQKFE | 1.75 | 0.26 |
| PGEEEQKFE | 2.00 | 0.00 |
| GEEEQKFEV | 1.96 | 0.07 |
| EEQKF | n.m. | |
| EEQKFEVE | n.m. | |
| EEQKFEVEK 356 | n.m. | |
| RVVPGEEEQKFEVEK 342              356 | Bindungsbereich | |

Abbildung 1a

Peptidscan monoklonale Antikörper
Klon 17-134/11 und 17-134/20

Abbildung 1b

Peptidscan monoklonale Antikörper
.Klon 17-134/11 und 17/134/20

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CELL STRUCTURE AND FUNCTION Band 10, Nr. 3, 1985, Seiten 195-208, Tokio, JP; K. TAKAHASHI et al.: "A Monoclonal Antibody Against Human Urokinase: The Epitope Structure and Sequence Homology with a Human Tissue-Type Plasminogen Activator" * Zusammenfassung; Seite 199; Seite 203-206 * --- | 1,6-10 | C 12 P 21/08 G 01 N 33/573 C 07 K 7/04 |
| Y | WO-A-8 403 564 (COMMONWEALTH SERUM LABORATORIES COMMISSION) * Zusammenfassung; Seite 2, Zeile 13 - Seite 8, Zeile 25; Ansprüche * --- | 1,2,6-10 | |
| Y | EP-A-0 190 711 (KOWA COMPANY, LTD.) * Zusammenfassung; Seite 8, Zeile 23 - Seite 13, Zeile 8; Seite 15, Zeile 20 - Seite 17, Zeile 4; Ansprüche * --- | 1,2,6-10 | |
| A | PATENT ABSTRACTS OF JAPAN Band 10, Nr. 345 (C-386), 20. November 1986; & JP-A.61148200 (FUJISAWA PHARMACEUT. CO LTD.) 05.07.1986 --- | 1,6,8,10 | RECHERCHIERTE SACHGEBIETE (Int. Cl.5) C 12 P 21/08 G 01 N 33/573 G 01 N 33/86 G 01 N 33/577 |
| Y | THROMBOSIS AND HAEMOSTASIS Band 53, Nr. 1, 1985, Seiten 45-50; I.R. MACGREGOR et al.: "Characterisation of Epitopes on Human Tissue Plasminogen Activator Recognised by a Group of Monoclonal Antibodies". * ganzes Dokument * --- -/- | 1,2,6-10 | C 07 K 7/04 C 07 K 7/08 C 12 N 15/58 C 12 N 9/72 |

Der vorliegende Recherchenbericht wurde für alle Patentansprüche erstellt

| Recherchenort | Abschlußdatum der Recherche | Prüfer |
|---|---|---|
| BERLIN | 09-07-1990 | JULIA P. |

**Europäisches Patentamt**

# EUROPÄISCHER RECHERCHENBERICHT

Nummer der Anmeldung

EP 90 10 6568

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 044 710 (SCRIPPS CLINIC AND RESEARCH FOUNDATION) * Zusammenfassung; Seite 15, Zeile 29 - Seite 16, Zeile 25; Seite 17, Zeile 30 - Seite 18, Zeile 31; Ansprüche 1,2,4-13 * --- | 1,2,6-10 | |
| A | EP-A-0 298 783 (YAMANOUCHI PHARMACEUTICAL CO. LTD.) * Zusammenfassung; Seite 3, Zeile 57 - Seite 4, Zeile 2; Seite 7, Zeilen 10-13; Seite 8, Zeile 20 - Seite 10, Zeile 52; Anspruch 8 * ----- | 1,6,8,10 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| BERLIN | 09-07-1990 | JULIA P. |

EPO FORM 1503 03.82 (P0403)